# EUROPEAN PATENT SPECIFICATION

(11) **EP 0 624 250 B1**
(45) Date of publication and mention of the grant of the patent: **23.04.2003**
(21) Application number: 93904606.6
(22) Date of filing: 25.01.1993
(51) Int. Cl.: G01N 30/02, G01N 33/22, G01N 33/26, G01M 3/20, G01N 33/28

(54) **METHOD OF IDENTIFYING CHEMICALS BY USE OF NON-RADIOACTIVE ISOTOPES**
VERFAHREN ZUR IDENTIFIZIERUNG VON CHEMIKALIEN DURCH NICHT-RADIOAKTIVE ISOTOPE
PROCEDE D'IDENTIFICATION DE PRODUITS CHIMIQUES A L'AIDE D'ISOTOPES NON RADIOACTIFS

(30) Priority: 29.01.1992 US 825478
(43) Date of publication of application: 17.11.1994
(73) Proprietor: ISOTAG TECHNOLOGY, INC., North Miami Beach, Florida 33181 (US)
(72) Inventor: Anderson, David King, Houston, Texas 77069 (US); Gonzales, Manuel Edu, Kingswood, Texas 77345 (US); Valenti, Nicholas Paul, Kingswood, Texas 77339 (US)
(74) Representative: Howden, Christopher Andrew
(86) International application number: PCT/US93/00647
(87) International publication number: WO 93/015398

(56) References cited:
- EP-A- 0 149 125
- US-A- 4 520 109
- US-A- 4 764 474
- Advanced Recovery Week, Volume 1, No. 37, D. JANECKY, "Subsurface Liquid Flow Tracing Enhanced by Los Alamos", pages 1 and 9.

## Description

### BACKGROUND OF THE INVENTION

Contamination of water with potentially hazardous materials is a common problem facing industry, the government and the general public. As a result of spills in waterways, leakage from storage facilities and surface discharges, contaminants are slowly destroying our water supply. Such contaminants may further enter our water supplies via subsurface soil and/or rock formations and eventually percolate into the groundwater. There are over two hundred organic and inorganic chemicals which have been identified in various groundwater supplies alone. Such ground water is the principal source of municipal water. agricultural irrigation. and water used by industry. There is thus a consistent health threat to our drinking water supplies. In addition, chemical discharging into intercoastal waters has resulted in damage to marine life as well as to marine ecosystems.

It is a fairly common occurrence to find such contaminants in our lakes and rivers as well as the surrounding oceans. The amount of unlawful dumping of such wastes is increasing in the waters of the United States. Groundwater, drinking water and waste water continues to be jeopardized as such activities continue. Useful methods of ascertaining the source of such pollutants into our waterways is essential.

Clearly there is a longfelt need by the public for a safe technique for "serializing" or "fingerprinting" petroleum, petroleum products and bulk chemicals in storage or transit so that responsibility for dumping, spilling or leakage of such chemicals can be appropriately determined. There is also a need for serializing bulk adulterants such as cyanides which are sometimes placed in foods and medicines by disturbed people to aid in apprehending such people. There is also a longfelt need by the petroleum and chemical industry for safe techniques to serialize oil and other chemical products for internally auditing the transfer of such products to prevent and/or prove theft.

It has been proposed to use radioactive materials as tracers in fluids. However, the use of radioactive materials for fingerprinting liquids would not be totally satisfactory. The consumption of petroleum products containing radioactive tracers, for example, would result in their uncontrolled release into the environment. It has also been proposed to use certain non-radioactive tracers in reservoir characterization studies to determine fluid residence times and conductive fluid flow paths. However, in such applications, the tracer is detected in salt water. Salt water is a very simple chemical composition and it is easy to obtain a low detection threshold because there are not many interfering materials. However, our proposals to label crude oil and other chemical materials with low levels of non-radioactive tagging agents have been met with skepticism because of the presumed difficulties in detecting such tagging agents. We have found that it is not difficult to identify many non-radioactive materials at very low levels if one knows what one is looking for.

### SUMMARY OF THE INVENTION

According to the invention, there is provided a method of tagging a fluid as described in claim 1.

The invention, makes it possible for a party to prove lack of responsibility for a material found at a dump, spill or leak site. The materical can be analyzed to obtain analysis results which identify the presence or absence of tagging agents in the material. Then, based on such analysis results, the party establishes either that said party never had custody of said material or that although said party had custody of said material at one time. such custody had been passed from said party to a transferee.

### DETAILED DESCRIPTION OF THE INVENTION

The invention pertains to a method for identifying the source of a transported chemical shipment. The method may be employed to verify that a sample received by an individual is identical to the sample that was shipped. In addition, the invention may be employed to detect the source of a newly introduced contaminant in a source, such as a water supply.

The method employs a non-radioactive chemical isotope which, with the material being transported, is introduced into the storage container prior to the container being loaded onto a freight vessel. Non-radioactive organic compounds are employed. The amount of isotopic compound introduced into the storage vessel may be less than one part per 10⁹ parts (ppb) of the chemical being transported. For certain isotopic compounds. the amount of isotopic compound introduced is between about 1 to about 5 ppb of the chemical being transported.

The invention finds particular applicability for marking chemical samples. Marking of the sample permits the recipient of the cargoed product to verify that the sample received is identical to the sample that was shipped. In this embodiment of the invention, the non-radioactive isotope substance is admixed with the chemical to be transported prior to shipment of the chemical. Upon arrival at its destination point, the chemical shipment is analyzed. Matching the isotopic compound with the isotopic compound introduced into the storage vessel prior to shipment is indicative that the shipped chemical is identical to the chemical received. The invention has applicability in the shipment of any chemical commodity, regardless of method of shipping or chemical structure of the commodity.

The method has particular applicability in the shipment of crude oil, refined oil, grains, processed and unprocessed chemicals as well as with bulk refined products. In addition, the invention may be employed in the shipment of a pollutant, hazardous material or a toxic material. As such, the invention has particular applicability in the identification of spilled shipments of spilled oil, pesticides, cyanide based compounds, arsenic containing compounds, dioxin, military chemical agents, military biological agents, naphthalene and biphenols.

The chemical substance may be a non-radioactive isotope of the chemical shipment being transported.

The chemical substance is a non-radioactive isotope of such organic solvents as acetone, acetonitrile, benzene, bromobenzene, chlorobenzene, chloroform, cyclohexane, dichlorobenzene. trichloroethylene, diethylether. diglyme, dimethylsulfoxide, dioxane, ethanol, methanol, methylene chloride, nitrobenzene, octane, pyridine, tetrachloroethane, tetrahydrofuran, tetrametholsilane, toluene, trifluoroacetic acid, trifluoroethyl alcohol, xylene, ammonium bromide, or acetyl chloride.

The organic compounds used are those which have been deuterated, i.e., wherein the hydrogen atoms covalently bound to carbon atoms are replaced with deuterium atoms. Deuterium is a non-radioactive isotope of hydrogen which is often called heavy hydrogen. Deuteration of organic compounds can be accomplished by methods known in the art such as those disclosed in U.S. Pat Nos. 3,746,634 and 3,876,521 wherein deuteration is effected with deuterium gas in the presence of a Group VII or VIII metal catalyst at a temperature between about 100 and about 300 degrees C. The non-radioactive isotopes for use in this invention may further be prepared in accordance with the prior art teachings of such materials used in the medical arts.

The non-radioactive chemical substance may have the heavy atom in any position of the molecule. Likewise, one or more of the reactive sites of a molecule may contain a heavy atom. For example, the number of permutations possible with n-octane is in the thousands since one or all of the hydrogen atoms of the molecule may be substituted with deuterium as set forth below:
CH₂DCH₂CH₂CH₂CH₂CH₂CH₂CH₃;
CH₃CHDCH₂CH₂CH₂CH₂CH₂CH₃;
CH₂DCHDCH₂CH₂CH₂CH₂CH₂CH₃;
CH₂DCH₂CH₂CH₂CH₂CH₂CH₂CH₂D;
CH₂DCHDCHDCH₂CH₂CH₂CH₂CH₃;
CH₂DCHDCHDCH₂CH₂CH₂CH₂CH₂D.
The number of uniquely identifiable combinations of deuterated n-octanes naturally decreases the chance that more than one shipping vessel will contain the same non-radioactive isotope.

When the tagging agents employed are selected from a moderately sized collection of tagging agents, the use of two or more tagging agents makes possible a very large number of unique custody tag possibilities.

The method of this invention may further be employed for the identification of source of non-radioactive materials infiltrating water supplies. The method has particular applicability where the substance infiltrating the water supply is environmentally toxic and hazardous. By this process, a non-radioactive isotope of a hydrogen containing organic chemical substance is introduced into a storage vessel containing the chemical supply to be transported prior to loading of the storage vessel onto the freight cargo leaving the exit port When spills of the transported chemical are suspected, a sample of contaminated water is recovered from the water supply. The sample is then analyzed. Detection of the non-radioactive isotope in the contaminated sample is indicative of the source of the spill. The hydrogen containing organic chemical substance may be a non-radioactive isotope of the chemical shipment being transported.

Suitable as the chemical substance used in the detection of the polluting source are those set forth above.

Still further the method of this invention may be employed to identify the source of chemical leakage from a land-based storage tank containing such a contaminant. The situs of the leakage may be either a water body or terrain. The presence of the contaminant is effectuated by recovering a sample of the contaminated area and analyzing the sample for the presence of the non-radioactive isotope to determine the location of the particular land-based storage tank which is leaking.

Isotopic identification may be readily achieved by mass spectroscopy, nuclear magnetic resonance spectroscopy or gas chromatography analysis. For instance, the spectra or retention time of the labelled isotope [prior to being introduced into the vessel containing the desired (ordered) chemical] may be compared to the spectra or retention time of the contaminant present in the water supply. See further B. B. McInteer et al., "The ICONS Facility: Separating Nitrogen and Oxygen Isotopes at Los Alamos", Los Alamos Technical Bulletin, March 1988.

Preferred embodiments of the invention, fluid compositions can be labeled or tagged by incorporating custody tag or custody tag modifiers therein. The fluid compositions are generally gases or liquids. The liquids are generally classified as oil based or water based. Oil based liquids generally include petroleum, and petroleum products. Volatile custody tags and custody tag modifiers are used to tag gases. Oil soluble custody tags and custody tag modifiers are generally used to tag oil based liquids. Water soluble custody tags and custody tag modifiers are generally used to label water based liquids. The custody tags are integral with the fluids and are nearly impossible to remove.

The amount of custody tag or tag modifier incorporated into the fluid can vary over a wide range. However, the tagging agent should always be added in an amount sufficient to be detected in the tagged product. Because the labeled fluids may be diluted a great deal with other materials before a sample is taken for analysis it can be desirable to incorporate a relatively large amount of custody tag into the fluid, although the amount used will generally be below about 1 ppm for economic reasons. On the other end of the scale, using current technology, certain tagging agents can probably be detected at levels of a few parts per trillion in gases and simple water solutions. The amount of custody tag employed in oil based liquids will generally be between these two ranges. Often, the custody tag will be used in an oil based liquid at below the 500 ppb level. In many instances, a concentration of custody tag in the range of 1 to 100 ppb will give desirable results.

The custody tag can be added to the fluid using a variety of techniques, depending on how well dispersion is expected. For example, the custody tag can be metered into a stream as it flows through a line. This will generally provide a better result than simply dumping the custody tag into a large storage tank. for example. However, an oil tanker can be treated by pouring the custody tag in the hold and then filling the tanker with oil. It is preferred to add the custody tag continuous to the fluid through a method system at a transfer or storage facility.

A custody tag will comprise at least two tagging agents, preferably three or more. Custody tag modifiers comprise at least one tagging agent, preferably only one. A custody tag modifier can be used to relabel a fluid containing a custody tag to indicate, for example, a transfer of custody. Preferably, the tagging agent present in a custody tag modifier is different from any of the tagging agents in the custody tagged fluid being relabeled.

Tagging agents suitable for use can generally be described as non-radioactive hydrogen containing organic compounds which are not naturally occurring and which are identifiable in tagged fluids at low thresholds of detection. Besides the tagging agents listed above, another example of suitable materials is the class of halogenated hydrocarbons, such as chlorinated and/or fluorinated alkenes, alkanes and aromatics. These materials are easily detected at low concentrations using gas chromatography techniques coupled with ion traps and/or mass spectrometer techniques. Preferably, the tagging agents used can be detected in the fluid which contains them at concentrations of less than 500 ppb, such as in the 1-100 ppb range. It is desirable to assemble a library or collection of suitable tagging agents and make selections from said library to formulate custody tags based on compatibility of the tagging agents with the fluid to be tagged and the use of a unique tag. Compatibility is rather easy to determine and is based on the range of properties of the fluid to be transported or stored. It does not require an especially large collection of tagging agents to accomplish the capability to provide unique combinations. For example, 1,000 tagging agents can be used to formulate over 41 x 10⁹ unique 3-component custody tags. Where the goal is to police dumps. spills and leaks, a record should be made of the custody tags which have been assigned to individual companies or shipments. The records should be gathered or complied into a database. The database can be referred to in the event of a spill, leak or dump to assign responsibility.

The presently preferred analysis technique for the detection of tagging agents utilizes a gas chromatograph coupled with a mass spectrometer although other chromatographic techniques can be used as well. It is first necessary, of course, to obtain a sample of the material to be analyzed for the presence of tagging agents. The sample is formed into a gas chromatograph stream and the stream is then flowed through the gas chromatograph. Predetermined portions of the stream are trapped and analyzed for tagging agent. Generally speaking, the analysis is carried out with a mass spectrometer. For difficult separations, the trapped portions of the sample are formed into a second stream and flowed through a second gas chromatograph. Predetermined portions of the second gas stream are trapped and analyzed for tagging agent. The determination of which portions of the chromatograph stream to trap is generally made before the original analysis of the sample and is usually based on retention time. It is made using knowledge of the tagging agent collection from which the tagging agents were selected, sometimes after a calibration run using known combinations of tagging agents from the collection.

Besides product tracking, one of the more important uses of the invention is expected to be in proving the innocence of environmental wrongdoing. Suppose a company is suspected or accused of contributing to the amount of noxious materials present at the site of a dump, spill or leak. Proving lack of culpability would be much easier with the use of custody tags.

A sample of the material should first be obtained and then analyzed to determine whether any tagging agents are present. If no tagging agents were founds the company should be able to establish Jack of culpability if it could show that it routinely used tagging agents during the time period in question. If tagging agents were found, the company should be able to establish Jack of culpability if it used different tagging agents than those that were found, or if it required its transferees of the material to use the tagging agents that were found.

## Claims

1. A method of tagging a fluid to render it distinguishable from other fluids, comprising introducing into said fluid at least two taggants each of which will disperse in said fluid, each taggant being a different deuterated version of the same hydrogen-containing organic chemical compound and wherein, in each deuterated version, in a significantly larger proportion of the molecules thereof than is to be expected from the natural abundance of deuterium, at least one hydrogen site in the moleude is occupied by deuterium, and wherein, in each said taggant, the same hydrogen site or combination of hydrogen sites is occupied by deuterium as in the other molecules of that taggant and a different hydrogen site or combination of hydrogen sites is occupied by deuterium than in the other taggants.

2. A method according to claim 1 wherein a said taggant comprises deuterated octane, or deuterated n-octene or deuterated acetone.

## Patentansprüche

1. Verfahren zur Markierung eines Fluids, um es von anderen Fluids unterscheidbar zu machen, welches umfaßt, daß in besagtes Fluid wenigstens zwei Markierungsmittel eingebracht werden, von denen jedes in besagtem Fluid dispergieren wird, wobei jedes Markierungsmittel eine unterschiedliche deuterierte Version derselben wasserstoffhaltigen organischen chemischen Verbindung ist, und wobei, in jeder deuterierten Version, in einem signifikant größeren Anteil der Moleküle derselben, als von der natürlichen Häufigkeit von Deuterium zu erwarten ist, wenigstens eine Wasserstoffstelle im Molekül von Deuterium besetzt ist, und wobei, in jedem besagten Markierungsmittel, dieselbe Wasserstoffstelle oder Kombination von Wasserstoffstellen von Deuterium besetzt ist wie in den anderen Molekülen dieses Markierungsmittels und eine unterschiedliche Wasserstoffstelle oder Kombination von Wasserstoffstellen von Deuterium besetzt ist als in den anderen Markierungsmitteln.

2. Verfahren nach Anspruch 1, **dadurch gekennzeichnet, daß** ein besagtes Markierungsmittel deuteriertes Octan oder deuteriertes n-Octan oder deuteriertes Aceton umfaßt.

## Revendications

1. Procédé pour marquer un fluide pour le rendre distinguable d'autres fluides, comprenant l'introduction dans ledit fluide d'au moins deux marqueurs, où chacun va se disperser dans ledit fluide, chaque marqueur étant une version deutériée différente du même composé chimique organique hydrogéné et dans lequel, dans chaque version deutériée, dans une proportion de ses molécules significativement supérieure à ce qui est prévu à partir de l'abondance naturelle du deutérium, au moins un site hydrogène dans la molécule est occupé par du deutérium, et dans lequel, dans chaque dit marqueur, le même site hydrogène ou la même combinaison de sites hydrogène est occupé par du deutérium comme dans les autres molécules de ce marqueur, et un site hydrogène ou une combinaison de sites hydrogène différent est occupé par du deutérium différemment que dans les autres marqueurs.

2. Procédé selon la revendication 1, dans lequel un dit marqueur comprend de l'octane deutérié, ou du n-octane deutérié, ou de l'acétone deutériée.
